# EUROPÄISCHE PATENTSCHRIFT

(11) **EP 4 251 109 B1**
(45) Veröffentlichungstag und Bekanntmachung des Hinweises auf die Patenterteilung: **23.09.2026**
(21) Anmeldenummer: 21824295.6
(22) Anmeldetag: 25.11.2021
(51) Int. Cl.: A61F 11/20, A61M 25/01, A61M 25/00

(54) **OHRKATHETER UND EINFÜHRHILFE FÜR KATHETER**
EAR CATHETER AND INSERTION AID FOR CATHETER
CATHÉTER AURICULAIRE ET ÉLÉMENT D'AIDE À L'INSERTION POUR CATHÉTER

(30) Priorität: 27.11.2020 DE 102020131572; 02.03.2021 DE 102021105036
(43) Veröffentlichungstag der Anmeldung: 04.10.2023
(73) Patentinhaber: Auriventis Medical GmbH, 79540 Lörrach (DE)
(72) Erfinder: SUDHOFF, Holger, 33604 Bielefeld (DE); SUDHOFF, Maximilian, 33604 Bielefeld (DE); OBRADOVIC, Aleksandar, 79539 Lörrach (DE); OBRADOVIC, Milisav, 79539 Lörrach (DE)
(74) Vertreter: Schneiders & Behrendt Bochum
(86) Internationale Anmeldenummer: PCT/EP2021/082967
(87) Internationale Veröffentlichungsnummer: WO 2022/112402

(56) Entgegenhaltungen:
- EP-A1- 2 781 193
- US-A1- 2006 106 361
- US-A1- 2015 231 369
- US-A1- 2016 058 983
- US-A1- 2017 119 414
- US-A1- 2019 209 815
- US-B2- 9 604 041

## Beschreibung

Die Erfindung betrifft einen Ohrkatheter mit einer Abdichtung zur Applikation von Medikamenten in die Ohrtrompete und eine Einführhilfe für Katheter mit einem Kopfstück, einem Mittelstück und einem Endstück, wobei die Einführhilfe aus einem körperverträglichen Polymer besteht. Die Einführhilfe dient insbesondere dazu, die Platzierung eines Ohrkatheters durch die Nase eines Patienten in das Mittelohr zu erleichtern.

Es sind zahlreiche Erkrankungen des Mittel- und Innenohrs bekannt, die eine örtliche Behandlung erfordern. Soweit eine topische Behandlung durchgeführt werden muss, kann diese zum einen durch das Trommelfell und zum anderen über die Ohrtrompete durchgeführt werden. Auch die Behandlung des Innenohrs kann über den Mittelohrbereich erfolgen. Häufig wird zur Behandlung ein durch die Nase eingeführter Ohrkatheter eingesetzt, auch um Medikamente einzuführen. Allerdings ist insbesondere die Verabreichung von Medikamenten in flüssiger Form in die Ohrtrompete oder das Mittelohr häufig nicht nachhaltig, da das Medikament kurzfristig durch die Ohrtrompete in den Nasenrachenraum ablaufen kann.

Eine Reihe von Infektionen, Entzündungen, aber auch die Behandlung eines Hörsturzes oder der Menière-Krankheit erfordern eine längerfristige Behandlung, die sich über Tage oder wenige Wochen hinziehen kann. In der Regel wird in diesem Fall ständig neues Medikament, etwa ein Corticosteroid, zugeführt. Diese Behandlung ist zeitaufwändig und verlangt eine regelmäßige und dauerhafte ärztliche Überwachung und Betreuung.

Aus diesem Grunde wäre es wünschenswert, über einen Ohrkatheter zu verfügen, mit dem ein Medikament in das Mittelohr appliziert und dort über längere Zeit vorrätig gehalten werden kann. Eine solche Applikation soll nach Möglichkeit nicht-invasiv erfolgen, also ohne Verletzung des Trommelfells. In diesem Fall kommt allein der natürliche Zugang über den Nasenrachenraum und die Ohrtrompete (die Eustachi'sche Röhre) in Frage.

DE 10 2018 102 937 A1 beschreibt einen entsprechenden Ohrkatheter, der über den Nasenrachenraum in die Ohrtrompete einführbar ist und zur Abdichtung einen expandierbaren Ballon umfasst. Ein Injektionskanal ist durch den Ballon geführt, sodass distal des Ballons ein Medikament in die Ohrtrompete appliziert werden kann, dessen Rückfluss in den Nasenrachenraum durch den Ballon verhindert wird.

Der bekannte Ohrkatheter hat verschiedene Nachteile. Einerseits verfügt der Katheter nicht über einen Druckausgleich distal des Ballons, der den durch die Applikation eines Mediums entstehenden Überdruck im Mittelohr ausgleichen könnte. Ferner verfügt der bekannte Ohrkatheter über keinerlei Marker, die dem Anwender eine genaue Positionsbestimmung des eingeführten Katheters erlauben würden. Zudem kann ein Ballon, wie er als Dichtelement vorgeschlagen wird, gegebenenfalls zu Druckstellen führen. Dies lässt sich auch kaum durch eine gegebenenfalls vorgesehene Druckbegrenzung des Ballons und der Auswahl eines sehr anpassungsfähigen Ballons verhindern, da zumindest ein gewisser Druck angelegt sein muss, um eine abdichtende Wirkung des Ballons zu erreichen. Zudem ist die Handhabung eines Ohrkatheters mit einem inflatierbaren Ballon relativ kompliziert, da immer ein zusätzliches Instrument zur Inflation (und Deflation) des Ballons benötigt wird.

Die Anlage eines Ohrkatheters durch die Nase eines Patienten ist, insbesondere dann, wenn der Katheter aus einem sehr flexiblen Material besteht, nicht einfach. Eine Einführhilfe wäre hier wünschenswert. Problematisch gestaltet sich die Verwendung einer Einführhilfe allerdings dann, wenn ein Katheter für eine Behandlung, auch über einen längeren Zeitraum, in dem Mittelohr oder in der Ohrtrompete fixiert werden muss, da proximal in der Regel eine Vorrichtung zur Verbindung des Katheters mit einer weiteren Vorrichtung vorgesehen ist, etwa mit einer Infusionsflasche oder einer Vorrichtung zur Beaufschlagung mit Druck. In diesem Fall kann die Einführhilfe nicht einfach nach proximal über den Katheterschlauch abgezogen werden, da sich hier der Anschluss für eine weitere Vorrichtung oder eine weitere Vorrichtung an sich befindet. Ein Abziehen der Einführvorrichtung nach distal ist ebenso unmöglich, da der Katheter mit seinem distalen Ende in der Zielstruktur, wie beispielsweise der Ohrtrompete, festgelegt ist.

US 2019 209815 A1 offenbart einen Katheter zum Einbringen eines Mediums in das Mittelohr mit einem Schaft, einem aufblasbaren ersten Ballon, einem aufblasbaren zweiten Ballon, einem Applikationslumen und einem Entlüftungslumen.

US 2006 106361 A1 betrifft eine implantierbare Verabreichungsvorrichtung mit einem Reservoir mit einer Substanz, das an einem Verabreichungskatheter oder einem Verabreichungsinstrument befestigt ist und in den Körper einführbar und an einer gewünschten Stelle positionierbar ist. Die Substanz wird aus dem Reservoir mit einer Geschwindigkeit abgegeben, die die gewünschte diagnostische oder therapeutische Wirkung hervorruft.

US 2017 119414 A1 offenbart ein System zum Dilatieren einer Eustachischen Röhre mit einem Führungselement und einem Dilatationskatheter, wobei der Dilatationskatheter relativ zum Führungselement verschiebbar ist. Der Dilatationskatheter umfasst einen Schaft, ein distal am Schaft angeordnetes expandierbares Element und einen Aktuator, wobei das expandierbare Element so konfiguriert ist, dass es durch Betätigung des Aktuators in eine expandierte Konfiguration übergeht.

Aufgabe der Erfindung ist es somit, einen Ohrkatheter mit einer Abdichtung zur Verfügung zu stellen, der zumindest einige der benannten Nachteile nicht aufweist, einfach in der Handhabung ist und insbesondere auch bei längerer Verweildauer nicht oder nur in einem geringen Maß zu Irritationen in der Ohrtrompete führt.

Die Aufgabe wird durch eine Vorrichtung gemäß Anspruch 1 gelöst. Weitere Ausgestaltungen der Erfindung zeigen die abhängigen Ansprüche auf.

Die Aufgabe wird durch einen erfindungsgemäßen Ohrkatheter gemäß Anspruch 1 gelöst, der einen Katheterschlauch mit einem Injektionslumen zur Einbringung von Medikamenten und mit einem Druckentlastungslumen zur Druckentlastung im Mittelohr sowie ein expandierbares Dichtelement umfasst.

Ein solcher Ohrkatheter kann nicht-invasiv durch die Nase und den Nasenrachenraum in die Ohrtrompete eingeführt und dort so platziert werden, dass das Dichtelement nach erfolgter Expansion die Ohrtrompete zum Rachenraum hin flüssigkeitsdicht abdichtet. Das Dichtelement dichtet dabei die Verbindung vom distal zum Dichtelement gelegenen Mittelohrbereich hin zum proximal des Dichtelements gelegenen Nasenrachenraum so ab, dass die durch das distal des Dichtelements endende Injektionslumen eingebrachten Flüssigkeiten praktisch nicht mehr in den Nasenrachenraum abfließen können.

"Distal" bezeichnet im Zusammenhang mit der vorliegenden Erfindung den entlang des Katheters beziehungsweise der Einführhilfe vom Benutzer wegweisenden Teil der Vorrichtung, während "proximal" den entlang des Katheters beziehungsweise der Einführhilfe zum Benutzer hinweisenden Teil bezeichnet. Weitere Einzelheiten zu den Lagebeziehungen lassen sich den Figuren entnehmen.

Als Dichtelement ist erfindungsgemäß ein expandierbarer Schirm vorgesehen.

Der Injektionskanal verläuft durchgehend vom distalen bis zum proximalen Ende des Katheterschlauchs und endet distal hinter dem Dichtelement.

Das Druckentlastungslumen beginnt ebenfalls am proximalen Ende des Katheterschlauchs und endet distal hinter dem Dichtelement. Die genaue Lage der distalen Öffnung des Druckentlastungslumens distal des Dichtelements kann variieren, funktionell wichtig ist lediglich, dass die distale Austrittsöffnung auch distal des Dichtelements liegt.

Es können weitere Lumen im Katheterschlauch vorgesehen sein, beispielsweise ein Lumen als weiteres Injektionslumen oder ein Lumen beispielsweise zur Aufnahme eines entfernbaren Stützdrahtes zur Stabilisierung des Katheterschlauchs während der Platzierung.

Neben diesen Lumen umfasst der Katheterschlauch zumindest ein weiteres Lumen als Inflationslumen, wenn als Dichtelement ein inflatierbarer Ballon vorgesehen ist.

Für den Katheterschlauch können übliche Materialien verwendet werden, wie sie aus der Kathetertechnik bekannt sind. Es handelt sich dabei in der Regel um Kunststoffe, die medizinisch zugelassen sind, beispielsweise PVC, Polyethylen, Polypropylen und dergleichen. Die Materialien sollten hinreichend flexibel sein, um das Einführen des Katheters in die Ohrtrompete zu erlauben.

Der erfindungsgemäße Schirm umfasst selbstexpandierbare Stützelemente, die mit einer Membran bedeckt sind, wobei der Schirm in seiner Gesamtheit reversibel von einem komprimierten in einen expandierten Zustand überführbar ist. Dies ermöglicht eine gegebenenfalls erforderliche Re-Platzierung des Schirms.

Die selbstexpandierbaren Stützelemente können als einzelne, unverbundene Stützelemente vorliegen, beispielsweise als eine Vielzahl ringförmiger oder speichenartiger Segmente. Vorzugsweise sind die selbstexpandierbaren Stützelemente jedoch zumindest teilweise miteinander verbunden und in Form eines Gerüsts, beispielsweise in der Art einer Gefäßstütze, eines Stents, vorgesehen. Entsprechend können die selbstexpandierbaren Stützelemente beziehungsweise das Gerüst durch Laserschneidverfahren aus einem Rohr oder in Form einer geflochtenen Struktur vorgesehen sein.

Als Materialien für das Gerüst bieten sich bekannte Formgedächtnismaterialien an, wie sie auch von sonstigen selbstexpandierbaren Implantaten bekannt sind. Dies sind insbesondere Federstahle, Nitinol-Verbindungen oder Polymere. Die Materialien für das Schirmgerüst sollten jedoch nicht resorbierbar sein, um die Stabilität des Schirms nicht zu gefährden.

Da der Schirm des erfindungsgemäßen Ohrkatheters lediglich dem Abdichten und nicht wie sonstige Gefäßstützen dem Offenhalten eines Lumens dient, ist nur eine geringe Radialkraft des Schirms erforderlich. Hohe Radialkräfte sind sogar zu vermeiden, da diese zu Druckstellen und Nekrosen in der empfindlichen Ohrtrompete führen können. Die bekannten Materialien lassen eine sehr genaue Einstellung der Radialkräfte zu.

Die selbstexpandierbaren Stützelemente beziehungsweise das Gerüst sind von einer Membran überzogen. Die Membran ist flüssigkeitsundurchlässig. Als Materialien für die Membran bieten sich entsprechende flüssigkeitsundurchlässige, biokompatible Folien an, wie sie auch von sonstigen Implantaten mit Membranen - beispielsweise von Stentgrafts - bekannt sind. Insbesondere geeignet sind verschiedene Kunststoffmembranen, beispielsweise aus PTFE oder ePTFE.

Die Membran ist sicher an dem Gerüst zu befestigen, was auf unterschiedliche Weise, beispielsweise durch Verkleben, Verschweißen oder durch geeignete Klemmvorrichtungen erfolgen kann. Kombinationen dieser Techniken sind denkbar. Die Verbindung zwischen Gerüst und Membran muss zuverlässig sein, sie ist jedoch nicht solchen Belastungen ausgesetzt wie beispielsweise bei Stentgrafts, da der Schirm des Ohrkatheters nicht einem ständigen Blutfluss ausgesetzt ist, sondern lediglich dem Zurückhalten einer applizierten Flüssigkeit dient.

Vorzugsweise ist die Membran mit ihrem proximalen Abschnitt flüssigkeitsdicht mit dem distalen Katheterende verbunden, wie dies nachfolgend detailliert für den Schirm beschrieben wird.

Der Schirm ist vorzugsweise konisch bzw. trichterförmig aufgebaut mit einem proximalen Ende mit einem ersten Durchmesser und einem distalen Ende mit einem zweiten Durchmesser, wobei der zweite Durchmesser größer ist als der erste Durchmesser.

Es ist jedoch eine Vielzahl weiterer Formen des Schirms beziehungsweise des Schirmgerüsts denkbar, die nachfolgend anhand ausführlicher Figuren noch näher beschrieben werden.

Der Schirm ist mit seinem proximalen Ende am distalen Ende des Katheterschlauchs festgelegt. Der erste Durchmesser des Schirms ist entsprechend so zu wählen, dass dieser eine entsprechende Verbindung mit dem Katheterschlauch ermöglicht, im Wesentlichen also dem Durchmesser des Katheterschlauchs entspricht.

Vorzugsweise kann am proximalen Ende des Schirms eine Befestigungshilfe beispielsweise in Form eines rohr- oder schlauchförmigen Fortsatzes vorgesehen sein, der sich innerhalb des distalen Katheterlumens oder darum herum anbringen lässt und so eine flüssigkeitsdichte Verbindung zwischen Schirm und Katheterschlauch ermöglicht. Der Durchmesser der Befestigungshilfe ist entsprechend des Durchmessers des Katheters zu wählen, wobei der Außendurchmesser der Befestigungshilf insbesondere dem Innendurchmesser des Katheters entspricht oder umgekehrt.

Weitere Verbindungen zwischen Schirm und Katheter sind denkbar. Hierzu gehören insbesondere Schraubverbindungen, Einrastverbindungen, Formschlussverbindungen, Klebverbindungen oder Spritzgussverbindungen, um nur einige der Möglichkeiten zu nennen.

Die Anbringungsstelle des Schirms am Katheterschlauch darf dabei die Expansion beziehungsweise Kompression des Schirms durch die noch genauer zu beschreibende Expansionsvorrichtung nicht behindern. Entsprechend ist eine Anbringung des Schirms auf der Außenseite des Katheterschlauchs eher zu vermeiden beziehungsweise ist eine Anbringung des Schirms zu vermeiden, die zu einer Kante an der Außenseite des Katheters führt.

Vorzugsweise wird der Schirm an der Innenseite des distalen Endes des Katheterschlauchs oder mit dem Katheterende abschließend, also an der distalen Austrittsöffnung des Katheterschlauchs, so an dem Katheterschlauch angebracht, dass die Lumen der Austrittsöffnungen, insbesondere die des Injektionslumens und des Druckentlastungslumens, nicht verschlossen werden und das Applizieren einer Flüssigkeit und die Ableitung eines Überdrucks weiterhin möglich sind. Gleiches gilt für das Inflationslumen sofern als Dichtelement ein Ballon vorgesehen ist.

In einer alternativen Ausführungsform der Variante mit einem Schirm als Dichtelement können die Lumen in einem verlängerten Abschnitt des Katheterschlauchs auch distal über das distale Ende des Schirms hinausragen. Zur Vermeidung von Verletzungen ist insbesondere bei dieser Ausführungsform und der Ausführungsform mit einem Ballon eine atraumatische Spitze an distalen Ende des so verlängerten Katheterschlauchs vorgesehen.

Der zweite Durchmesser des Schirms beziehungsweise der normative Durchmesser des Ballons sind so zu wählen, dass diese mindestens dem Durchmesser der Ohrtrompete an der Stelle entsprechen, die sie flüssigkeitsdicht abschließen sollen. Vorzugsweise werden der zweite Durchmesser des Schirms beziehungsweise der normative Durchmesser des Ballons etwas größer als der entsprechende Durchmesser der Ohrtrompete gewählt.

Die Konstruktion des Schirms aus Gerüst und Membran ist so vorgesehen, dass sich die nachfolgend noch genauer zu beschreibende Expansionsvorrichtung problemlos sowohl nach proximal von dem komprimierten Schirm zurückziehen, als auch nach distal über den expandierten Schirm vorschieben lässt.

Hierzu bietet es sich an, den Schirm trichterförmig bzw. konisch/kegelförmig zu gestalten, also mit einer sich von proximal nach distal aufweitenden Struktur. Zumindest sollte der Schirm an dem Ende, das den an den Katheterschlauch anschließt, einen entsprechenden sich aufweitenden Bereich umfassen.

Die Form des Schirms im frei expandierten Zustand, also in einem Zustand ohne äußere Zwänge weder durch die Expansionsvorrichtung noch durch beispielsweise die Ohrtrompete, kann von der Membran und/oder dem Gerüst bestimmt werden. Entsprechend begrenzt an der jeweiligen Stelle entweder die Membran die maximal mögliche Expansion des Schirms oder das Gerüst beziehungsweise die Stützelemente.

Der Schirm und der Katheterschlauch sind flüssigkeitsdicht miteinander verbunden, sodass eine durch den Katheterschlauch applizierte Flüssigkeit nicht zwischen Katheterschlauch und expandiertem Schirm nach proximal entweichen kann.

Der Schirm wird erfindungsgemäß mithilfe der Expansionsvorrichtung reversibel von einem komprimierten in einen expandierten Zustand überführt. Die Expansionsvorrichtung ist hierzu - wie auch der Katheterschlauch - schlauchförmig vorgesehen und umschließt den Katheterschlauch weitestgehend formschlüssig, wobei eine flüssigkeitsdichte Abdichtung zwischen Expansionsvorrichtung und Katheterschlauch nicht erforderlich ist. Die Dichtigkeit des Ohrkatheters wird allein durch den Schirm bestimmt.

Die Expansionsvorrichtung ist zumindest in axialer Richtung beweglich gegenüber dem Katheterschlauch angeordnet, sodass eine Verschiebung der Expansionsvorrichtung entlang des Katheterschlauchs von proximal nach distal bzw. in umgekehrter Richtung möglich ist. Die Expansionsvorrichtung erstreckt sich vorzugsweise vom proximalen Ende des Katheterschlauchs bis maximal hin zur Ansatzstelle des Schirms am Katheterschlauch und lässt sich bis über das distale Ende des Schirms hin entlang des Katheterschlauchs verschieben.

Eine Verschiebung der Expansionsvorrichtung nach distal führt zu einer Kompression des Schirms, wenn sich nämlich die Expansionsvorrichtung über den am distalen Ende des Katheterschlauchs festgelegten Schirm schiebt. Eine Verschiebung der Expansionsvorrichtung nach proximal führt entsprechend zu einer Expansion des Schirms, wenn sich nämlich die Expansionsvorrichtung von Schirm herunterschiebt und sich der Schirm dann ohne äußeren Zwang der Expansionsvorrichtung öffnen kann.

Zur besseren Handhabung umfasst die Expansionsvorrichtung an ihrem proximalen Ende ein optionales Bedienelement, mit dessen Hilfe die Expansionsvorrichtung entlang des Katheters verschiebbar ist.

Der Schlauch des Ohrkatheters sollte in etwa eine Länge von 15 cm bis 30 cm aufweisen. Die Länge sollte ausreichen, um den Katheter durch ein Nasenloch und den Nasenrachenraum in die Ohrtrompete einzuführen, wobei das proximale Ende im Wangenbereich des Patienten verbleibt oder in einer alternativen Ausführungsform auch hinter dem entsprechenden Ohr des Patienten fixierbar ist.

Der Ohrkatheter bzw. das Injektionslumen sind am proximalen Ende mit einer Anschlussvorrichtung für übliche Spritzen versehen, die das Befüllen des Ohrkatheters mit Druckmedium und das Einführen von Medikamentenlösung in das Innenohr erlauben. Bei der Anschlussvorrichtung handelt es sich zweckmäßigerweise um das in der Medizin übliche Luer-Lock-System, an das mit entsprechenden Einrichtungen verschiedene Spritzen angekuppelt werden können.

Entsprechend kann auch das Druckentlastungslumen am proximalen Ende des Katheters mit einer Anschlussvorrichtung versehen sein, es kann jedoch auch ohne spezielle Anschlussvorrichtung enden. Bei der Anschlussvorrichtung handelt es sich zweckmäßigerweise um das in der Medizin übliche Luer-Lock-System, an das mit entsprechenden Einrichtungen verschiedene Spritzen angekuppelt werden können.

Vorzugsweise lässt sich die Expansionsvorrichtung im expandierten Zustand des Schirms am proximalen Ende des Katheterschlauchs lösbar festlegen, um ein unbeabsichtigtes Komprimieren des Schirms zu verhindern. Vorzugsweise ist hierfür eine lösbare Verbindungsmöglichkeit zwischen dem Bedienelement der Expansionsvorrichtung und dem Luer-Lock-System vorgesehen. Vorstellbar sind hier einfache Einrastelemente, die zueinander kompatibel an dem Luer-Lock-System und dem Bedienelement vorgesehen sind. Weitere Einrastvorrichtungen sind denkbar, dem Fachmann sind hier verschiedenste Lösungen bekannt.

Um während der Platzierung des Ohrkatheters durch die Nase und den Nasenrachenraum eine unbeabsichtigte Expansion durch Verrutschen der Expansionsvorrichtung nach proximal zu verhindern, kann zwischen der Anschlussvorrichtung an proximalen Ende des Ohrkatheters und der Bedienhilfe der Expansionsvorrichtung ein Abstandselement vorgesehen sein, das zweckmäßigerweise als geschlitztes Rohr vorgesehen sein und so abgezogen und wieder aufsteckt werden kann.

Es versteht sich, dass der Ohrkatheter bzw. das Injektionslumen eine Verschlussvorrichtung aufweist, die einen Rückfluss des applizierten Medikaments nach dem Befüllen zurückhält. Entsprechend weist das Injektionslumen entweder an seinem distalen oder an seinem proximalen Ende einen Verschluss, beispielsweise ein Einwegventil, auf, der das Abfließen des injizierten Medikaments durch das Injektionslumen verhindert. Bei einem Einwegventil öffnet die Austrittsöffnung entsprechend durch den Druck der Injektion und schließt danach wieder.

Auch das Druckentlastungslumen weist entweder an seinem distalen oder an seinem proximalen Ende einen Verschluss, beispielsweise ein Einwegventil, auf, der das Abfließen des injizierten Medikaments durch das Druckentlastungslumen verhindert. Bei einem Einwegventil öffnet die Austrittsöffnung entsprechend durch den Druck und schließt danach wieder.

Die Verschlussvorrichtung kann als einfaches selbstschließendes Ventil vorgesehen sein.

Es ist vorteilhaft, den Katheter mit einer Handhabungshilfe für die Steuerung bei der Platzierung auszustatten. Des Weiteren ist es vorteilhaft, am Katheter ein gekrümmtes distales Ende vorzusehen, das die Steuerung und Positionierung des Katheters in die Ohrtrompete hinein und in der Ohrtrompete erleichtert. Ein solches gekrümmtes Ende wird bei Kathetern und Führungsdrähten als "Pig-Tail" bezeichnet und ist weithin üblich.

Der erfindungsgemäße Ohrkatheter wird nicht-invasiv durch die Nase und den Nasenrachenraum in die Ohrtrompete eingeführt. Die Einführung kann ohrmikroskopisch überwacht werden. Sobald das expandierbare Dichtelement, also der Schirm oder der Ballon, den zu verschließenden Teil der Ohrtrompete erreicht hat, wird er durch Betätigen der Expansionsvorrichtung expandiert.

Zur Unterstützung bei der Positionsbestimmung des Katheters beziehungsweise des Dichtelements können Markierungen am distalen Ende des Katheters jedoch proximal des Dichtelements vorgesehen sein. Solche Markierungen, kurz Marker, können sequentiell vorgesehen sein und endoskopisch durch den Behandler während der Platzierung wahrgenommen werden. Vorteilhaft ist die Anbringung mehrerer Marker, die beispielsweise einen ersten unkritischen Bereich des Vorschubs markieren, beispielsweise mit einer grünen Markierung, einen zweiten Bereich erhöhter Aufmerksamkeit, beispielsweise mit einer gelben Markierung, und einen dritten kritischen Bereich, beispielsweise mit einer roten Markierung. Die Begriffe "unkritisch", "erhöhte Aufmerksamkeit" und "kritisch" beziehen sich dabei auf ein fortschreitendes Eindringen der Katheterspitze in das Innenohr und somit auf eine zunehmend kritische Nähe zu empfindlichen Bereichen.

Das Injektionslumen endet distal des Dichtelements. Am proximalen Ende weist es vorzugsweise einen Luer-Lock-Anschluss für eine Spritze auf, über die ein Medikament in den Mittelohrbereich appliziert werden kann.

Der erfindungsgemäße Ohrkatheter ist besonders geeignet zur Applikation von Antibiotikalösungen in den Mittelohrbereich, was bei hartnäckigen bakteriellen Infektionen sinnvoll ist. Auch kann die Standardbehandlung eines Hörsturzes, die mit einem Corticosteroid erfolgt, sinnvoll über einen solchen Ohrkatheter durchgeführt werden. Die Standardtherapie verlangt, dass ein solches Corticosteroid über längere Zeit appliziert wird, in der Regel etwa zwei Wochen, wobei die Applikation täglich neu vorgenommen werden muss, da das Medikament in den Nasenrachenraum hinein abläuft. Üblich ist eine Behandlungsdauer von 14 Tagen. Der erfindungsgemäße Ohrkatheter ermöglicht eine sehr viel intensivere und kürzere Behandlung, da er die ständige Präsenz des Medikaments gewährleistet. Die Behandlungsdauer verkürzt sich dadurch erheblich, unter Umständen auf wenige Tage. Er ist insbesondere geeignet, das Medikament über eine längere Zeit, insbesondere auch mehrere Tage, im Mittelohr zurückzuhalten.

Ferner können auch entzündungshemmende Medikamente über den Ohrkatheter eingeführt werden.

Der Ohrkatheter wird über eine der Nasenöffnungen gegebenenfalls unter Hinzunahme der erfindungsgemäßen Einführhilfe gelegt und kann nach der Expansion des Schirms und der Applikation des Medikaments mit einem geeigneten Pflaster auf der Wange oder alternativ hinter dem Ohr festgelegt werden.

Der Ohrkatheter verbleibt während der Behandlung in der Ohrtrompete und hält so das applizierte Medikament im Bereich der Ohrtrompete, ohne dass dieses in den Nasenrachenraum zurückfließen könnte. Der Patient bleibt somit mobil, ein längerer Aufenthalt in einer Klinik ist ebenso nicht erforderlich, eine regelmäßige ärztliche Überwachung reicht aus. Zur Überwachung kann die Ohrmikroskopie angewendet werden.

Nach der Behandlung kann der Ohrkatheter nach Kompression des Dichtelements problemlos entfernt werden.

Der Ohrkatheter erlaubt auch die Behandlung von Erkrankungen des Innenohrs, da die Medikamente in der Regel durch die Membran des runden Fensters zum Innenohr durchtreten können. Es handelt sich dabei um einen Diffusionsprozess.

Zu dem möglichen Indikationen gehören beispielsweise intratympanale Steroidgabe bei Hörsturz, intratympanale Steroidgabe bei hydropischer Ohrerkrankung (Menière), intratympanale Gentamicingabe bei hydropischer Ohrerkrankung, intratympanale Kontrastmittelgabe bei Verdacht auf hydropische Ohrerkrankung, intratympanale Applikation zur Gentherapie (CRISPR/Cas9) oder auch intratympanale topische Antibiose. Den Einsatz bei weiteren Indikationen liegt in der Entscheidung des behandelnden Arztes.

Mögliche Substanzen, die auf diesem Wege appliziert werden können, umfassen beispielsweise Dexamethason, Methylprednisolon, Gentamycin, Neomycin, Wachstumsfaktoren, BDNF, Neurothrophin-3, N-Acetyl-Cystein, Dextran, Rhodamin oder auch Gadolliniumsalz. Die Applikation weiterer Substanzen liegt in der Entscheidung des behandelnden Arztes.

Vorteilhafterweise wird der Ohrkatheter mit einer speziellen Einführhilfe platziert. Eine für den Einsatz mit dem erfindungsgemäßen Ohrkatheter geeignete Einführhilfe umfasst drei Abschnitte, nämlich ein Kopfstück, ein Mittelstück und ein Endstück, mit einer in Längsrichtung des jeweiligen Abschnitts verlaufenden Trennlinie, entlang zumindest einiger Abschnitt, sodass die entsprechenden Abschnitte der Einführhilfe längs geöffnet werden können.

Die Einführhilfe lässt sich zumindest teilweise von einem platzierten Katheter entfernen, indem bestimmte oder alle Abschnitte der Einführhilfe über eine oder mehrere längs verlaufende Trennlinien verfügen. Die Trennlinien können dabei über einzelne, mehrere oder alle Abschnitte der Einführhilfe verlaufen. Sie können untereinander fortlaufend ausgebildet sein, denkbar ist also auch eine durchgängige Trennlinie über mehrere oder alle Abschnitte, oder die Trennlinien können unterschiedlich in ihrer Lage und Ausführung pro Abschnitt vorgesehen sein.

Eine Trennlinie kann eine Schwächungslinie sein, bei der das Material der Einführhilfe an dieser Stelle eine vergleichsweise geringere Festigkeit aufweist. Eine solche Schwächungslinie kann beispielsweise durch eine Laserbehandlung erzeugt werden, wird aber zweckmäßigerweise mechanisch durch Einschnitte oder Perforationslinien erzeugt, die das Aufreißen entlang dieser Linie erleichtern.

Solche Einschnitte können sich beispielsweise über 40 bis 70 % der Wandstärke der Einführhilfe in diesem Abschnitt erstrecken. Erst durch Einwirkung des Anwenders wird in diesem Fall die Auftrennung vollzogen.

Eine Trennlinie kann aber auch eine bereits vorhandene Öffnung darstellen, bei der das Material an der entsprechenden Stelle bereits vollständig durchtrennt ist, beispielsweise bei einer mehrteiligen Fertigung des entsprechenden Teils. In diesem Fall wird die Auftrennung nicht erst durch den Anwender vollzogen. Vielmehr sind die einzelnen Teile durch verschiedene noch zu beschreibende Verbindungselemente verbunden.

Die Einführhilfe besteht im Wesentlichen aus einem eher steifen Material, in aller Regel einem Polymer. In der Medizintechnik geeignete Polymere sind bekannt, infrage kommen insbesondere thermoplastische Kunststoffe und thermoplastische Elastomere, etwa Polyetherblockamide (Pebax). Eine gewisse Flexibilität erleichtert die Einführung, eine zu große Flexibilität bedeutet aber den Verlust an Führungsfähigkeit.

In einer ersten bevorzugten Ausführungsform ist die Einführhilfe zur Verwendung mit einem Ohrkatheter vorgesehen. Das Kopfstück der Einführhilfe ist abgewinkelt, um eine möglichst einfache Platzierung in der Ohrtrompete zu ermöglichen. Mittelstück und Endstück sind im Wesentlichen gerade ausgeformt.

Diese drei Abschnitte haben übliche Maße, beispielsweise etwa 2,5 cm für das Kopfstück und etwa 14 cm für das Mittelstück. Kopfstück und Mittelstück werden komplett über die Nase des Patienten in den Körper eingeführt, das Endstück verbleibt außerhalb des Körpers. Es hat beispielsweise eine Länge von 15 cm.

Es versteht sich, dass das Material der Einführhilfe aus einem körperverträglichen Material besteht. Zweckmäßigerweise ist dieses Material transparent.

Das Kopfstück ist auf übliche Weise gegen den Verlauf des Mittelstücks abgewinkelt, beispielsweise um einen Winkel von 30° bis 80°, insbesondere etwa 70°.

Bei dieser ersten Ausführungsform der Einführhilfe verlaufen zwei Trennlinien über die Länge sowohl des Kopfstücks als auch des Mittelstücks, die es erlauben, diesen Teil der Einführhilfe aufzureißen. Das Endstück selbst ist aufgespalten, bildet also zwei Enden, die von dem Anwender ergriffen werden können, um die Einführhilfe bei gelegtem Katheter aus Mittelohr, Ohrtrompete und Nase herauszuziehen. Die Trennlinien des Kopfstücks und des Mittelstücks setzen sich proximal in den Trennlinien der beiden Hälften des Endstücks fort, sodass der Anwender die gesamte Einführhilfe über die freien Enden aufreißen und entfernen kann. Die gebrauchte Einführhilfe wird entsorgt.

Es sei darauf hingewiesen, dass "distal" in diesem Zusammenhang den zum Mittelohr hinweisenden Teil der Einführhilfe bezeichnet, während "proximal" den vom Mittelohr wegweisenden Teil bezeichnet.

Es hat sich herausgestellt, dass in Einzelfällen die Einführung des Ohrkatheters mit der Einführhilfe gemäß der ersten Ausführungsform zu einer Belastung des Übergangs des Mittelstücks zum Endstück führen kann, dergestalt, dass die Trennlinien anfangen aufzureißen. Um ein vorzeitiges Aufreißen zu vermeiden, ist es sinnvoll, dass proximale Ende des Mittelstücks mit einem Sicherungselement zu versehen, dass ein solches Einreißen vermeidet. Ein geeignetes Sicherungselement ist beispielsweise ein Ring aus Klebeband, das vom Anwender vor Entfernung der Einführhilfe abgerissen wird. Dazu weist es zweckmäßigerweise ein freies, nicht klebendes Ende auf, das das Entfernen erleichtert.

In der Praxis ergreift der Anwender nach Platzierung des Katheters die beiden freien Enden des Endstücks der Einführhilfe, zieht daran das Mittelstück und das Kopfstück über den Katheter aus dem Körper des Patienten heraus und reißt dabei entlang der Trennlinien die zusammenhängenden Teile zunächst am Mittelstück und dann am Kopfstück auf. Damit ist die Einführhilfe vom Katheter getrennt.

Bei der zweiten bevorzugten Ausführungsform eine Verwendung der Einführhilfe ebenfalls mit einem Ohrkatheter möglich. Hierfür kann das Kopfstück wie zuvor beschrieben abgewinkelt sein, um eine möglichst einfache Platzierung der Einführhilfe in der Ohrtrompete zu ermöglichen. Mittelstück und Endstück sind im Wesentlichen gerade ausgeformt.

Die sonstigen Ausführungen zur ersten Ausführungsform bezüglich der Dimensionen der Einführhilfe und der bevorzugten Materialien gelten hier ebenso.

Die zweite bevorzugte Ausführungsform unterscheidet sich von der ersten bevorzugten Ausführungsform vor allem durch den Mechanismus der Trennung der Einführhilfe vom Katheter. Bei der zweiten Ausführungsform erfolgt die Trennung der Einführhilfe vom Katheter insbesondere durch ein Aufklappen der Einführhilfe.

Entsprechend ist die Einführhilfe gemäß dieser zweiten Ausführungsform aufklappbar und vorzugsweise zweiteilig, nämlich der Länge nach in zwei Hälften teilbar, vorgesehen. Die Trennlinien sind wie zuvor beschrieben hier als durchgehende Schlitze vorgesehen. Die beiden Hälften sind vorzugsweise als spiegelbildlich identische Hälften vorgesehen. Es ist jedoch auch vorstellbar, dass die Einführhilfe nicht aus solchen spiegelbildlich identischen Hälften, sondern aus entsprechenden Dritteln oder Vierteln oder in sonstigen Verhältnissen zusammengesetzt ist. Bevorzugt sind jedoch spiegelbildlich identische Hälften.

Die Hälften sind vorzugsweise zumindest an einer Stelle dauerhaft miteinander verbunden, sodass sie beim Aufklappen entlang der Trennlinien nicht in zwei separate Teile auseinanderfallen. Ein entsprechendes Verbindungselement kann beispielsweise in Form eines Scharniers vorgesehen sein. Ein solches Scharnier ist vorzugsweise im Bereich des Endstücks vorgesehen und verbindet die beiden Hälften. Das Scharnier kann bereits bei der Herstellung der Bedienhilfe zwischen den beiden Hälften vorgesehen sein, beispielsweise in einem entsprechenden Spritzgussverfahren, oder auch nach Herstellung der beiden Hälften diese erst nachträglich verbinden.

Bei Ausführungsformen, die aus mehreren Längsteilen, so wie zuvor beschrieben, bestehen, sind entsprechend weitere Scharniere zwischen den Längsteilen vorgesehen.

Anstelle der Scharniere können auch sonstige Verbindungselemente vorgesehen sein, die die Längsteile der Einführhilfe verbinden. Solche Verbindungselemente müssen nicht zwingend eine dauerhafte Verbindung schaffen, es genügt, wenn die einzelnen Längsteile zuverlässig während der Platzierung des Katheters zusammengehalten werden.

Eine Kombination aus Scharnieren und sonstigen Verbindungselementen ist ebenso sinnvoll. So kann beispielsweise eine zuverlässige Verbindung der Einführhilfe durch zumindest ein Scharnier am Endstück erfolgen und weitere Verbindungselemente können zur Unterstützung an Mittel- und Endstück oder auch am Kopfstück vorgesehen sein.

Die weiteren Verbindungselemente können dabei beispielsweise Rast- oder Klickelemente sein, deren Gegenstücke sich an den jeweils zusammenzufügenden Längsteilen befinden. Die weiteren Verbindungselemente sind dann also vorzugsweise Teil der Einführhilfe.

Es sind jedoch auch weitere Verbindungselemente vorstellbar, beispielsweise in Form von Ringen, die an den entsprechenden Längsteilen der Einführhilfe vorgesehen sind und die zum Aufklappen der Einführhilfe gelöst werden können, beispielsweise an dafür vorgesehenen Sollbruchstellen. Weitere Verbindungselemente können auch in Form der zuvor beschriebenen Sicherungselemente vorgesehen sein.

Die zweite Ausführungsform der Einführhilfe ist aufgrund des bezüglich der wesentlichen Teile nicht destruktiven Aufklappmechanismus auch als Mehrwegartikel vorstellbar. Entsprechend kann insbesondere bei der Konstruktion des Endstücks eine massivere Bauart verfolgt werden, die eine besonders angenehme Haptik für den Verwender beispielsweise durch den Einsatz besonders ausgeformter Griffbereiche ermöglicht. Ebenso können in dem Endstück wiederverwendbare Verbindungselemente vorgesehen sein, beispielsweise in Form von Riegeln, die ein wiederholtes Öffnen und Verschließen des zusammengeklappten bzw. zusammengefügten Endstücks ermöglichen.

In einer alternativen Gestaltung der zweiten Ausführungsform sind nur das Mittel- und das Endstück aufklappbar vorgesehen. Das Kopfstück hingegen ist einteilig rohrförmig vorgesehen und an seinem proximalen Ende über ein Kopplungselement mit dem distalen Ende des Mittelstücks verbindbar.

Das Kopplungselement kann beispielsweise als Gewinde vorgesehen sein. Das Kopfstück verfügt entsprechend über ein Außen- oder Innengewinde und das Mittelstück über das entsprechende Gegenstück. Auch denkbar ist es, dass das Kopplungselement in Form eines separaten Bauteils vorliegt, nämlich als Gewindeelement, in das Kopf- und Mittelstück eingedreht werden, wobei auch hier die Anordnung von zueinander passenden Außen- und Innengewinden den jeweiligen Anforderungen entsprechend vom Fachmann ausgewählt wird. Bevorzugt ist eine Ausführungsform, bei der Kopf- und Mittelstück über ein Außengewinde verfügen und diese Außengewinde in das Innengewinde des Kopplungselements eingeschraubt werden.

Andere Kopplungselemente beispielsweise in Form von insbesondere kraftschlüssigen Klick- oder Rastelementen sind vorstellbar.

Das Kopplungselement dient hierbei zusätzlich als Verbindungselement, indem es die beiden Teile des Mittelstücks und des daran angeschlossenen Endstücks verbindet.

Bei der alternativen zweiten Ausführungsform der Einführhilfe bleibt somit das Kopfstück nach dem Entfernen von Mittel- und Endstück auf dem Katheter und kann nach einem Verschieben hin zum proximalen Katheterende beispielsweise als Fixierungshilfe genutzt werden, um das proximale Katheterende beispielsweise hinter dem Ohr des Patienten zu fixieren.

Die alternative zweite Ausführungsform der Einführhilfe bietet auch den Vorteil, dass das Kopfstück austauschbar ist. In einem entsprechenden Set könnten dann dem Grundelement einer solchen Einführhilfe, nämlich der Einheit aus Mittel- und Endstück, eine Vielzahl von Kopfstücken unterschiedlicher Winkel beigefügt werden. Aus diesen unterschiedlichen Kopfstücken kann der Anwender dann einen für die jeweilige Aufgabe das am besten geeignete Kopfstück auswählen. Denkbar sind hier die verschiedensten Variationen bezüglich Winkel des Kopfstücks, Länge des Kopfstücks oder auch die Materialien, die besonders stabil oder flexibel sein können.

Entsprechend können in einem solchen Set auch Kopfstücke vorgesehen sein, die nicht unbedingt Behandlungen des Ohrs betreffen und so auch für die Verwendung mit anderen Kathetern geeignet sind.

Somit kann die alternative zweite Ausführungsform der Einführhilfe auch als Kombinationsprodukt umfassend ein wiederverwendbares Grundelement mit austauschbaren und wegwerfbaren Kopfstücken vorgesehen sein.

Die zweite Ausführungsform kann alternativ an dem Kopf- und/oder Mittelstück zusätzlich eine schlauchartige Hülle umfassen, die diesen Abschnitten eng anliegt. Die Hülle gibt den zweiteiligen Kopf- und/oder Mittelstücken zusätzlichen Halt, indem sie die zwei Hälften dieser Bauelemente fest zusammenhält. Die Hülle kann einteilig oder mehrteilig vorgesehen sein und an verschiedenen Stellen des Kopf- und/oder Mittelstücks angebracht sein. Die Hülle kann auch nur ringförmig vorgesehen sein, also nicht als längerer oder durchgehender Schlauch.

Zum Öffnen und Entfernen der Hülle kann eine Perforationslinie vorgesehen sein. Eine solche Perforation dient jedoch vor allem dem Bedienungskomfort. Ohne entsprechende Perforation kann die Hülle vom Benutzer alternativ durch einen Längsschnitt oder durch Aufreißen entfernt werden. Das Material der Hülle kann entsprechend ausgewählt werden.

Da die Hülle bei der Behandlung mit eingeführt wird, sind hier insbesondere reibungsarme Materialien vorteilhaft. Dem Fachmann sind hier verschiedenste Materialien bekannt, wie verschiedene Kunststoffe, beispielsweise auch PTFE oder ePTFE.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen. Die Erfindung ist jedoch nicht auf die gezeigte Ausführungsvariante beschränkt.

Es zeigen:
- Figur 1: die Gesamtansicht einer ersten bevorzugten Ausführungsform des erfindungsgemäßen Ohrkatheters mit einem Schirm als Dichtelement.
- Figur 2: die Details des proximalen und distalen Abschnitts der bevorzugten Ausführungsform gemäß Figur 1.
- Figur 3: weitere Details des distalen Abschnitts einer weiteren bevorzugten Ausführungsform.
- Figur 4: verschiedene Umrisse möglicher Schirmgestaltungen.
- Figur 5: verschiedene Möglichkeiten der Verbindung des proximalen Schirmabschnitts mit dem Katheterschlauch.
- Figur 6: eine Übersicht einer bevorzugten Ausführungsform mit einem Ballon als Dichtelement, wobei diese Ausführungsform nicht unter die Ansprüche fällt, aber zum Verständnis der Erfindung beiträgt.
- Figur 7: die Details des proximalen Endes der Ausführungsform gemäß Figur 6, wobei diese Ausführungsform nicht unter die Ansprüche fällt, aber zum Verständnis der Erfindung beiträgt.
- Figur 8: weitere Details des proximalen Endes der Ausführungsform gemäß Figur 7, wobei diese Ausführungsform nicht unter die Ansprüche fällt, aber zum Verständnis der Erfindung beiträgt.
- Figur 9: weitere Details des proximalen Endes der Ausführungsform gemäß Figur 7 in einer Variante, wobei diese Ausführungsform nicht unter die Ansprüche fällt, aber zum Verständnis der Erfindung beiträgt.
- Figur 10: eine erste Ausführungsform der Einführhilfe.
- Figur 11: das Prinzip des Gebrauchs und der Entfernung der ersten Ausführungsform der Einführhilfe.
- Figur 12: der Verlauf einer Trennlinie der ersten Ausführungsform.
- Figur 13: eine erste Ausführungsform der Einführhilfe mit Aufreißsicherung.
- Figur 14: eine zweite Ausführungsform der Einführhilfe im aufgeklappten Zustand
- Figur 15: eine perspektivische Ansicht einer alternativen Bauform der zweiten Ausführungsform der Einführhilfe
- Figur 16: eine Aufsicht auf die Innenseite einer Hälfte der Einführhilfe gemäß Figur 14
- Figur 17: Detailansichten der Figur 16
- Figur 18: eine perspektivische Ansicht gemäß Figur 15 mit Hülle.
- Figur 19: einen Detailausschnitt der perspektivischen Ansicht gemäß Figur 18

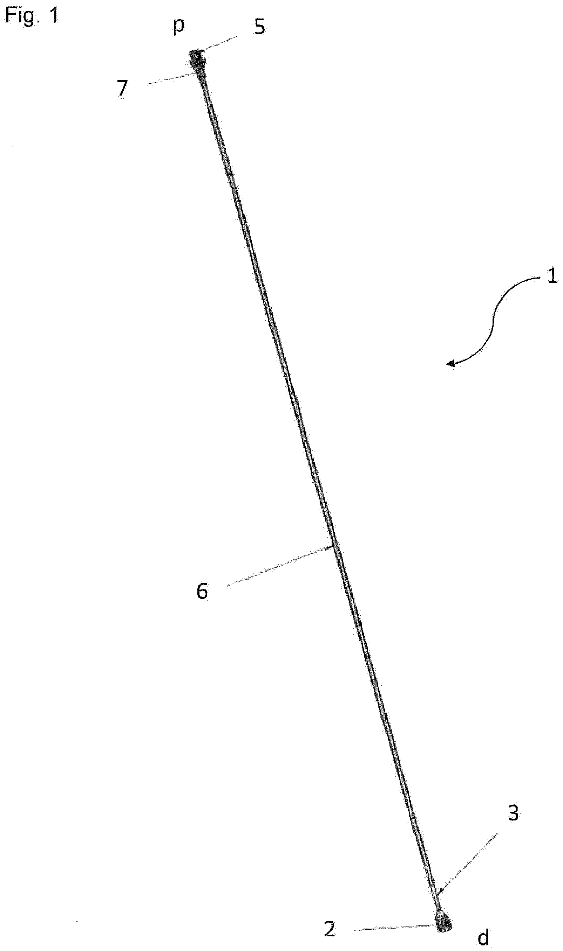

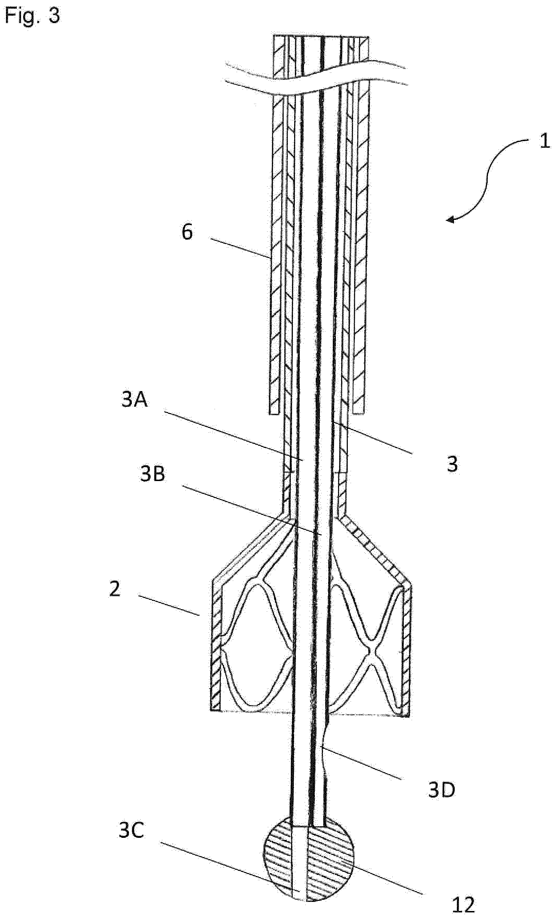

Der erfindungsgemäße Ohrkatheter 1 gemäß Figur 1 umfasst einen Katheterschlauch 3, an dessen distalen Ende d ein selbstexpandierbarer Schirm 2 vorgesehen ist. Am proximalen Ende p des Katheterschlauchs 3 befindet sich ein Luer-Lock-Anschluss 5 für eine herkömmliche Spritze, mit der das zu applizierende Medikament durch das Injektionslumen 3A eingeführt werden kann. Das proximale Ende kann weiterhin einen zusätzlichen Anschluss für das Druckentlastungslumen umfassen (nicht dargestellt). Das einzige Lumen des Katheters 3 umfasst bei dieser Ausführungsform das Injektionslumen 3A, bevorzugt ist jedoch eine Ausführungsform, bei der der Katheter neben dem Injektionslumen 3A ein weiteres Lumen zur Druckentlastung umfasst, wie in der nachfolgenden Figur 3 dargestellt..

Der Ohrkatheter 1 hat insgesamt eine Länge von etwa 15 cm bis 30 cm bei einem Außendurchmesser des Katheterschlauchs 3 von vorzugsweise 2 bis 3 mm und einem Innendurchmesser des Injektionslumens von etwa 1 mm.

Eine schlauchförmige Expansionsvorrichtung 6, die form- bzw. reibschlüssig um den Katheterschlauch 3 vorgesehen ist, dient zur Expansion bzw. Kompression des Schirms 2. Die Expansionsvorrichtung 6 ist wie auch der Katheterschlauch vorzugsweise aus Kunststoff gefertigt.

Am proximalen Ende p der Expansionsvorrichtung 6 ist eine Bedienhilfe 7 vorgesehen, die die Handhabung der Expansionsvorrichtung 6 vereinfacht, da sie einen sicheren Zugriff und somit eine sichere Steuerung der Expansionsvorrichtung 6 erlaubt.

Die Expansionsvorrichtung 6 dient der Expansion bzw. der Kompression des Schirms 2. Wird die Expansionsvorrichtung 6 nach distal d über den Schirm geschoben und dort belassen, so befindet sich der Schirm 2 im komprimierten Zustand, wird die Expansionsvorrichtung 6 nach proximal p vom Schirm 2 heruntergeschoben und dort belassen, so befindet sich der Schirm 2 im expandierten Zustand.

Um während der Platzierung des Ohrkatheters 1 durch die Nase und den Nasenrachenraum eine unbeabsichtigte Expansion des Schirms 2 durch Verrutschen der Expansionsvorrichtung 6 zu verhindern, kann zwischen dem Luer-Lock 5 an proximalen Ende p des Ohrkatheters 1 und der Bedienhilfe 7 der Expansionsvorrichtung 6 ein Abstandselement vorgesehen sein, das zweckmäßigerweise als geschlitztes Rohr vorgesehen sein und so abgezogen und wieder aufsteckt werden kann (nicht dargestellt).

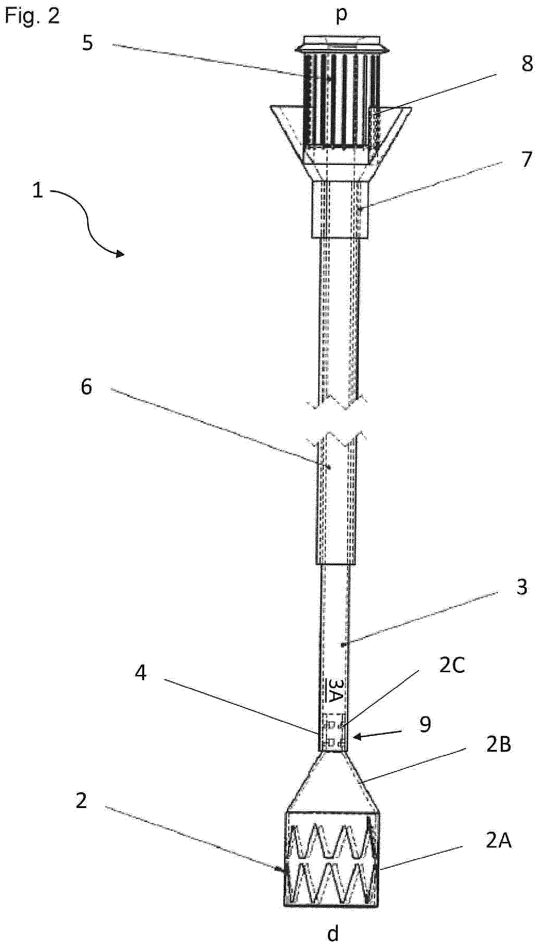

Figur 2 zeigt die Details des distalen Endes d und des proximalen Endes p des Ohrkatheters 1. Am distalen Ende d des Ohrkatheters 1 ist der selbstexpandierende Schirm 2 vorgesehen. Dieser ist vorzugsweise aus drei Abschnitten 2 A, B, C aufgebaut, nämlich einem distalen Abschnitt 2 A mit einem großen Durchmesser, der über eine bestimmte Länge zylinderförmig ohne nennenswerte Verjüngung verläuft und der im Wesentlichen den Teil des Schirms 2 darstellt, der einen flüssigkeitsdichten Kontakt mit der Wand der Ohrtrompete herstellt. Ein optionaler proximaler Abschnitt 2 C ist rohrförmig vorgesehen zur sicheren Festlegung des Schirms 2 im distalen Lumen des Katheterschlauchs 3.

Der offene Durchmesser des Katheterschlauchs 3, der das Injektionslumen 3A und das Druckentlastungslumen (nicht dargestellt) umfasst, soll sich dadurch nur unwesentlich verringern, um die Applikation von Flüssigkeiten und den Druckausgleich nicht zu behindern. Zwischen dem proximalen Abschnitt 2 C und dem distalen Abschnitt 2 A ist ein mittlerer Abschnitt 2 B vorgesehen, der trichter- bzw. konusförmig ausgestaltet ist und sich von dem kleineren Durchmesser des Abschnitts 2 C hin zum größeren Durchmesser des Abschnitts 2 A hin erweitert.

Der Aufbau des Schirms 2, also die Anteile der selbstexpandierenden Stützelemente bzw. des Gerüst einerseits und der Membran andererseits, kann über die Abschnitte 2 A bis C variieren. Vorzugsweise ist die Membran einteilig gefertigt, um eine bestmögliche Dichtigkeit zu gewährleisten, und erstreckt sich über alle Abschnitte 2 A bis C.

Um einen möglichst dichten Abschluss zwischen dem Schirm 2 und der Wandung der Ohrtrompete zu ermöglichen, kann distal ein zylindrischer Abschnitt 2 A über eine bestimmte Länge vorgesehen sein. Dieser Abschnitt umfasst neben der Membran auch eine Anzahl selbstexpandierender Stützelemente, die vorzugsweise als mäandrierende Ringsegmente vorgesehen sind und miteinander in Form eines Gerüsts oder als separate Stützelemente vorliegen können. Ein distaler Abschnitt 2 A ist jedoch optional, es sind auch Ausführungsformen denkbar, bei denen das distale Ende des konischen Abschnitts 2 B zugleich das distale Ende des Schirms 2 bildet.

Die Abschnitte 2 A bis C können insgesamt oder teilweise durch selbstexpandierende Stützelemente bzw. ein zusammenhängendes Gerüst gestützt werden. Die Struktur und das Design können in den einzelnen Abschnitten 2 A bis C variieren. So ist es am distalen Ende d des Schirms 2 wichtig, dass hier eine ausreichende jedoch nicht zu hohe Radialkraft realisiert wird, die einen möglichst dichten Abschluss zwischen Schirm 2 und Ohrtrompete gewährt, gleichzeitig jedoch nicht zu Druckstellen und Nekrosen führt. Entsprechend sind das distale Ende des Abschnitts 2 B und/oder der optionale Abschnitt 2 A zu gestalten. Der konische Abschnitt 2 B muss ansonsten keine entsprechenden Radialkräfte aufweisen, dient er doch vor allem der Unterbindung eines Rückflusses des Medikaments aus der Ohrtrompete in den Nasenrachenraum. Entsprechend wäre der Abschnitt 2 B bei Vorhandensein eines distalen Abschnitts 2 A grundsätzlich auch ohne Gerüststruktur oder entsprechende Stützelemente denkbar. Gleiches gilt für den Abschnitt 2 C, der letztendlich allein der Fixierung des Schirms 2 im Katheterschlauch 3 dient, die auch allein über eine Verbindung zwischen der Membran und dem Katheterschlauch 3 denkbar ist.

Es sind Ausführungsformen denkbar, bei denen der komplette Schirm 2 durch ein inneres Gerüst und/oder entsprechende Stützelemente und eine mit diesen verbundene Membran gebildet wird. Entsprechende Kombinationen aus Schirmabschnitten 2 A bis C, die neben der Membran auch ein Gerüst und/oder Stützelemente umfassen, findet der Fachmann ohne erfinderisches Zutun entsprechend den jeweiligen Anforderungen.

Die Expansionsvorrichtung 6, die schlauchförmig um den Katheterschlauch 3 vorgesehen ist, umfasst an ihrem proximalen Ende optional eine Bedienhilfe 7, die ein Vor- und Zurückschieben der Expansionsvorrichtung 6 erleichtert. In einer bevorzugten Ausführungsform kann eine Einrastvorrichtung 8 vorgesehen sein, mit der sich die Bedienhilfe 7 lösbar mit dem Luer-Lock-System 5 verbinden lässt, um ein Verschieben der Bedienhilfe 7 bei expandiertem Schirm 2 und insbesondere ein Komprimieren des Schirms 2 zu verhindern.

Die Expansionsvorrichtung 6 ist vorzugsweise schlauch- bzw. rohrförmig vorgesehen. Es sind auch Ausführungsformen denkbar, bei denen die Expansionsvorrichtung 6 zumindest teilweise geschlitzt vorgesehen ist, um beispielsweise die Flexibilität des Ohrkatheters 1 zu erhöhen.

Die Bedienhilfe 7 kann ringförmig vorgesehen sein, wobei auch hier geschlitzte Ausführungsformen oder Halteelemente an nur bestimmten Stellen der Expansionsvorrichtung 6 denkbar sind.

Um einen Rückfluss der applizierten Flüssigkeit durch das Injektionslumen 3A zu verhindern, ist vorzugsweise in dessen distalen Ende ein Einwegventil 4 vorgesehen. Ein entsprechendes Ventil ist für das Druckentlastungslumen vorgesehen (nicht dargestellt).

Bei der Behandlung wird nach Anlage des Katheters und Expansion des Schirms über eine Spritze das Medikament in das Mittelohr gefüllt und dort für die erforderliche Zeit belassen. Die Füllung des Mittelohrs wird ohrmikroskopisch überwacht. Die Prozedur wird bevorzugt unter örtlicher Betäubung durchgeführt, jedoch kann in besonderen Fällen eine Vollnarkose angeraten sein. Nach Applikation des Medikaments wird der proximale Teil des Applikationskatheters an der Wange mit einem Pflaster verklebt. Das Dichtelement verbleibt an Ort und Stelle in der Ohrtrompete und verhindert das Abfließen der injizierten Lösung. Nach Beendigung der Behandlung wird das Dichtelement komprimiert und der Katheter gezogen.

Figur 3 zeigt eine weitere bevorzugte Ausführungsform des Ohrkatheters 1 mit dem Injektionslumen 3A und dem Druckentlastungslumen 3B. Der Katheterschlauch 3 endet distal mit einer atraumatisch ausgeformten Spitze 12, wobei das Injektionslumen 3A distal der atraumatisch ausgeformten Spitze 12 mit einer Öffnung 3C endet und das Druckentlastungslumen 3B proximal der atraumatischen Spitze 12 mit einer Öffnung endet 3D. Im Unterschied zu der Ausführungsform gemäß Figur 2 wird zumindest ein Teil des Katheterschlauchs 3 mit Injektionslumen 3A und Druckentlastungslumen 3B durch dem Schirm 2 hindurchgeführt und endet erst distal des Schirms 2.

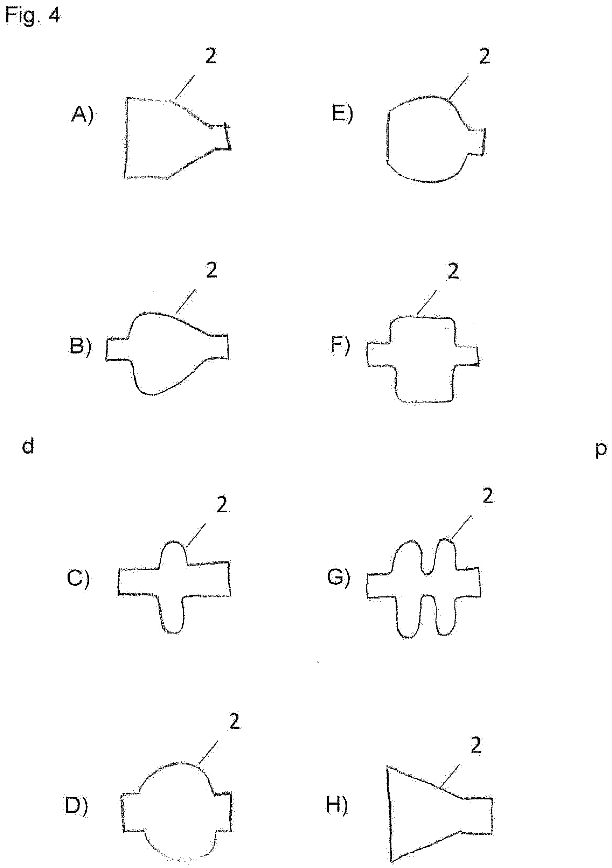

Figur 4 zeigt in den Abbildungen A bis H verschiedene mögliche Ausgestaltungen des Schirms 2 in Umrissdarstellungen, wobei links jeweils das distale Ende und rechts das proximale Ende liegt. Die Umrisse entsprechen jeweils der Kombination aus Gerüst und Membran, der Umriss des Schirms 2 kann also alternativ im Wesentlichen von dem Gerüst, der Membran oder der Kombination hieraus bestimmt sein.

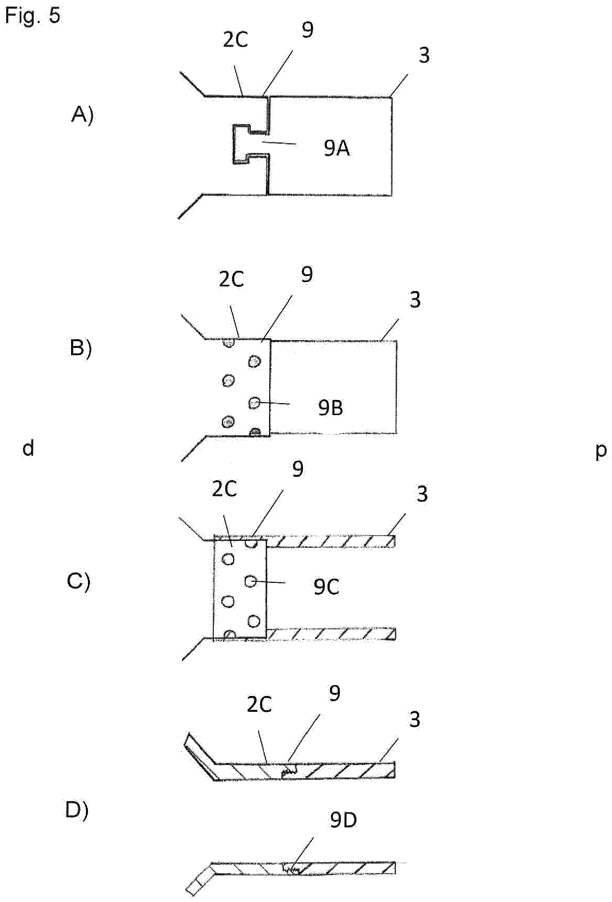

Figur 5 zeigt im Detail verschiedene Möglichkeiten der Verbindung 9 des proximalen Schirmabschnitts 2C und des Katheterschlauchs 3. Figur 5A zeigt eine Verbindung 9 mit Einrastelementen 9A, die vorzugsweise im Spritzgussverfahren verfestigt wird. Figur 5B zeigt eine Klebeverbindung 9, bei der der Katheterschlauch 3 in das proximale Ende des Schirms 2C reicht und mit diesem verklebt wird. Zur weiteren Stabilisierung der Klebverbindung 9 kann das proximale Schirmende 2C Ausstanzungen 9B umfassen. Alternativ kann diese Verbindung auch als reiner Formschluss erfolgen. Figur 5C zeigt eine Formschlussverbindung 9 mit optional zusätzlichen Halteelementen 9C, die den Reibungswiderstand erhöhen und so ein Herausgleiten des Schirms zusätzlich verhindern. Der Katheterschlauch 3 umgibt bei dieser Ausführungsform das distale Schirmende 2C. Diese Verbindung kann zusätzlich auch als Klebeverbindung vorgesehen sein. Figur 5D zeigt eine Schraubverbindung 9 mit einer Gewindeverbindung 9D, wobei das Außengewinde Teil des distalen Schirmendes 2C und das Innengewinde Teil des Katheterschlauchs 3 sein kann oder umgekehrt.

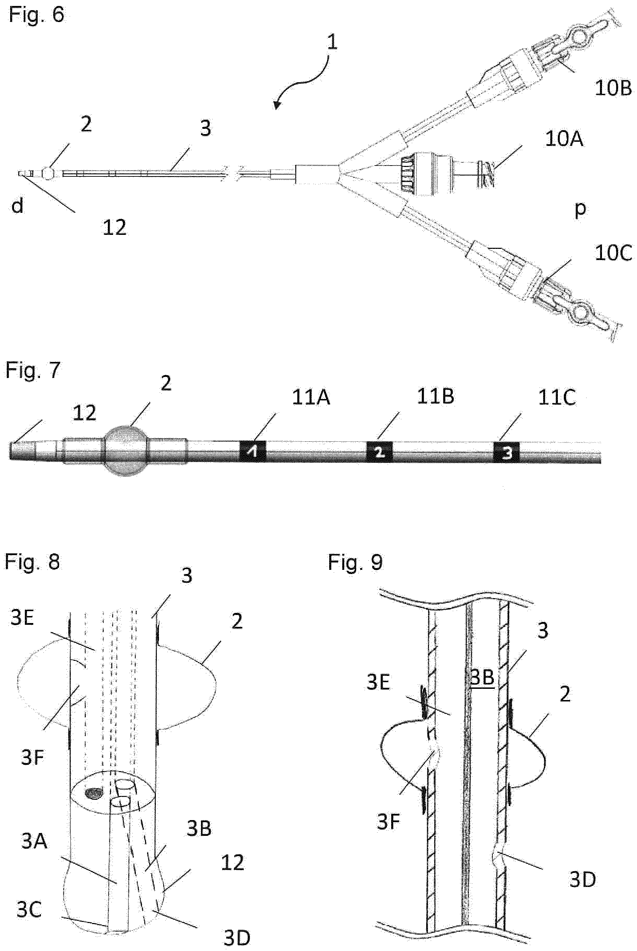

Figur 6 zeigt eine Komplettansicht einer Ausführungsform des Ohrkatheters 1 mit einem Ballon als Dichtelement 2, die nicht unter die Ansprüche fällt, aber zum Verständnis der Erfindung beiträgt. Am proximalen Ende des Katheters 3 sind drei Anschlüsse angeordnet, ein Anschluss 10B zur Inflation des Ballons, ein Anschluss 10A zum Anschluss einer Spritze an das Injektionslumen und ein Anschluss 10C zum Druckausgleich über das Druckentlastungslumen. Am distalen Ende des Katheters 1 ist eine atraumatische Spitze 12 vorgesehen.

Figur 7 zeigt eine Detailansicht des distalen Katheterabschnitts mit der atraumatischen Spitze 12, dem proximal davon gelegenen Ballon 2 und den drei proximal vor dem Ballon angebrachten Markern zu endoskopischen Bestimmung der Lage des Ohrkatheters während der Platzierung, wobei diese Ausführungsform nicht unter die Ansprüche fällt, aber zum Verständnis der Erfindung beiträgt.

Figur 8 zeigt eine Detailansicht einer ersten bevorzugten Ausführungsform des distalen Abschnitts des Katheterschlauchs 3, wobei das Injektionslumen 3A an der distalen Spitze der atraumatischen Spitze 12 mit einer Öffnung 3C endet und das Druckentlastungslumen 3B seitlich versetzt an der atraumatischen Spitze 12 mit einer Öffnung 3D endet. Das Inflationslumen 3E endet offen 3F im Bereich des Ballons 2, wobei diese Ausführungsform nicht unter die Ansprüche fällt, aber zum Verständnis der Erfindung beiträgt.

Figur 9 zeigt eine weitere bevorzugte Ausführungsform des distalen Abschnitts des Katheterschlauchs 3, wobei das Druckentlastungslumen 3B seitlich distal des Ballons 2 jedoch proximal der Spitze (nicht dargestellt) mit einer Öffnung 3D endet. Das Inflationslumen 3E endet im Bereich des Ballons 2 (das Injektionslumen ist hier dargestellt), wobei diese Ausführungsform nicht unter die Ansprüche fällt, aber zum Verständnis der Erfindung beiträgt.

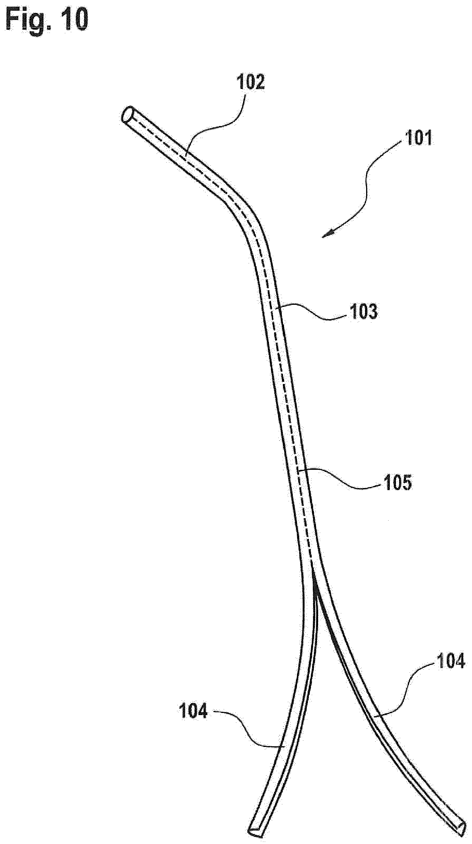

Figur 10 zeigt eine Einführhilfe 101 mit einem abgewinkelten Kopfstück 102, einem geraden Mittelstück 103 und dem in zwei Arme aufgespaltenen Endstück 104. Das Kopfstück 102 ist gegenüber dem Mittelstück 103 abgewinkelt, idealerweise um etwa 70° (anders dargestellt). Der Winkel von 70° ergibt sich gegenüber dem Verlauf des Mittelstücks 103.

Dass Endstück 104 ist in zwei Arme aufgespalten, die dem behandelnden Arzt als Handhabe für das Aufreißen der Einführhilfe 101 über die Länge dienen. In Fortsetzung der Aufspaltung erstreckt sich über die Länge des Mittelstücks 103 und des Endstücks 102 eine Trennlinie oder Naht 105, die als Schwächungszone ausgebildet sind, beispielsweise durch einen partiellen Einschnitt.

Die Einführhilfe 101 ist aus einem körperverträglichen Polymer gefertigt, dass eine hinreichende Steifigkeit für die verlangten Führungseigenschaften aufweist. Gleichzeitig darf das Polymer dem Aufreißprozess keinen zu großen Widerstand entgegenbringen. Infrage kommen übliche Polymere, wie Polyamid, Polypropylen oder auch Copolymere wie Pebax.

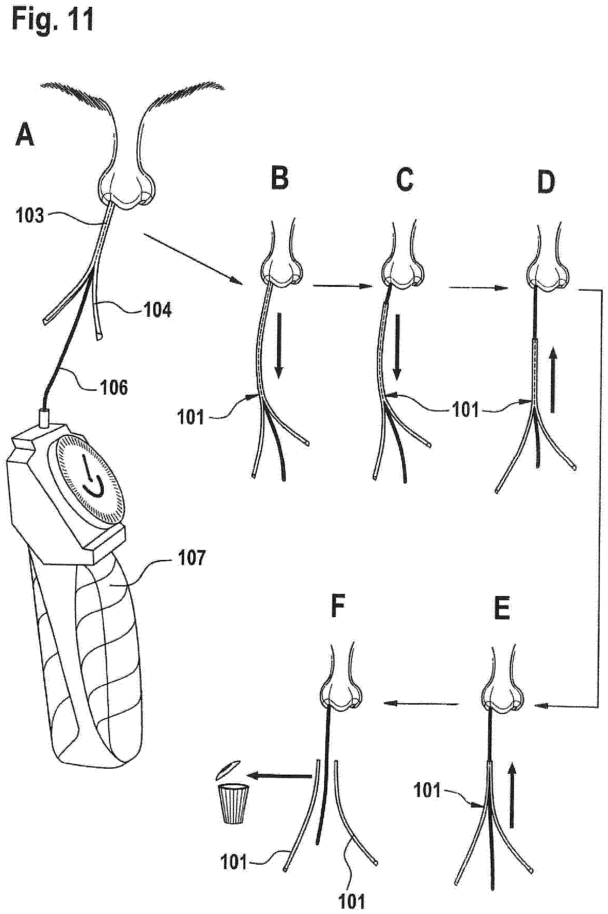

Figur 11 ist eine Abfolge, die in Bild A den durch die Nase eines Patienten durch die Einführhilfe 101 eingeführten Ohrkatheter 106 mit angeschlossenem Druckgenerator 107 zeigt, in Bild B die Entfernung der Einführhilfe 101 über den Katheterschlauch aus der Nase des Patienten, in Bild C die bereits vollständig aus der Nase des Patienten entfernte Einführhilfe 101, in Bild D die weithin aufgerissene Einführhilfe 101, in Bild E die bis zum distalen Kopfstück 102 aufgerissene Einführhilfe 101 und in Bild F die vollständig aufgerissene Einführhilfe 101, die zur Entsorgung bestimmt ist.

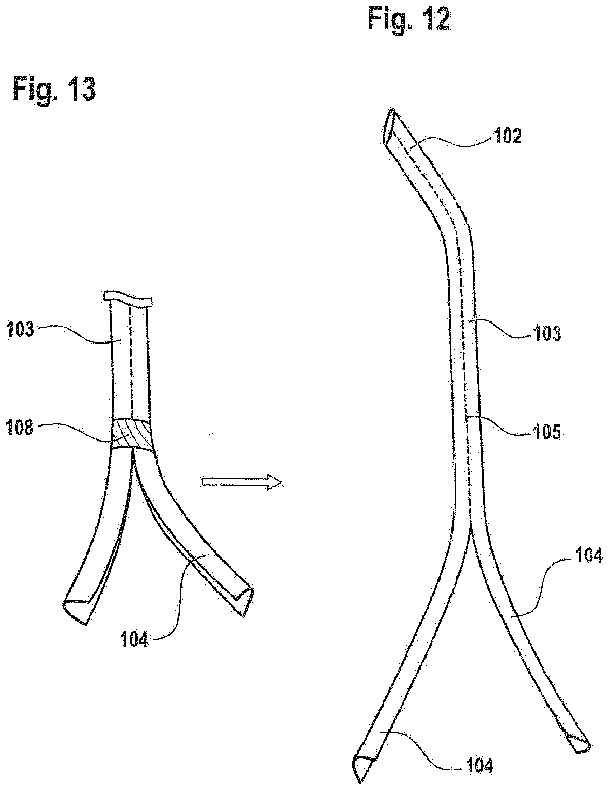

Figur 12 zeigt eine Einführhilfe 101 mit dem distalen Kopfstück 102, dem Mittelstück 103 und den beiden Armen des Endstücks 104 sowie der gestrichelt eingezeichneten Trennlinie 105. Die Trennlinie 105 setzt sich über die gesamte Länge des Mittelstücks 103 und des Endstücks 104 fort. Sie beginnt am Verbindungspunkt der beiden Arme des Endstücks 104. Es versteht sich, dass auf der gegenüberliegenden Seite eine entsprechende Trennlinie 105 vorhanden ist.

Figur 13 zeigt den proximalen Bereich einer Einführhilfe 101 mit einem Sicherungselement 108 am proximalen Ende des Mittelstücks 103. Das Sicherungselement 108 ist ein Klebestreifen, der die Einführhilfe 101 an bezeichneter Stelle umgibt und ein vorzeitiges Aufreißen entlang der Trennlinie 105 verhindert. Vor dem Aufreißen der Einführhilfe 101 wird der Klebestreifen 8 vom behandelnden Arzt abgerissen.

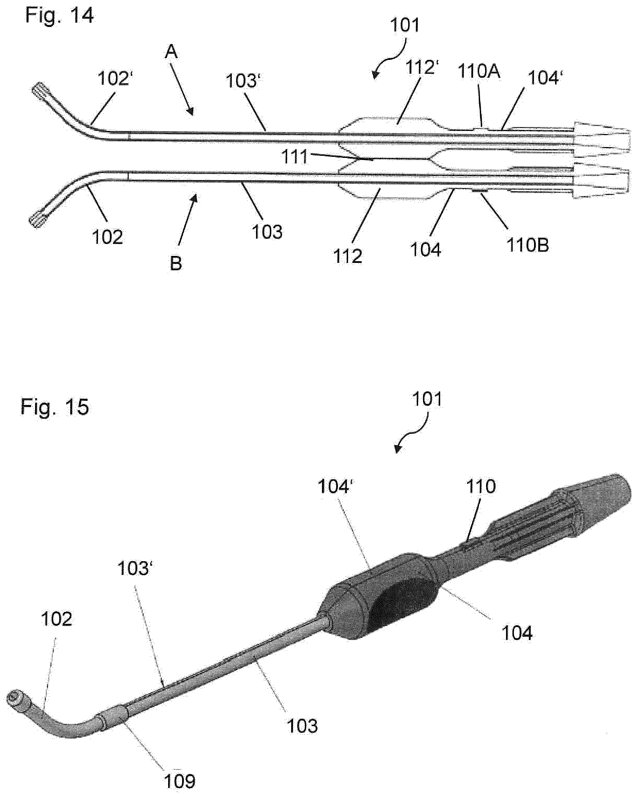

Figur 14 zeigt eine zweite Ausführungsform der Einführhilfe im aufgeklappten Zustand mit einer ersten Hälfte A umfassend eine Hälfte des Kopfstücks 102', eine Hälfte des Mittelstücks 103' und eine Hälfte des Endstücks 104' sowie einer zweiten Hälfte B umfassend eine Hälfte des Kopfstücks 102, eine Hälfte des Mittelstücks 103 und eine Hälfte des Endstücks 104. Die Hälften A, B der Einführhilfe 101 sind in diesem Beispiel spiegelbildlich identisch zueinander ausgeformt.

Das Kopfstück 102, 102' ist gegenüber dem Mittelstück 103, 103' abgewinkelt, bei der Verwendung als Einführhilfe für einen Ohrkatheter vorzugsweise um etwa 70° (hier anders dargestellt). Der Winkel von 70° ergibt sich gegenüber dem Verlauf des Mittelstücks 103, 103'. Ein Winkel des Kopfstücks 102, 102' gegenüber dem Mittelstück 103, 103' von 0° würde somit einem insgesamt gerade Verlauf von Mittel- und Kopfstück 103, 103', 102, 102' entsprechen.

Die Hälften A, B sind in einem Bereich des Endstücks 104, 104', vorzugsweise in einem Griffbereich 112, 112', miteinander über ein Verbindungselement 111 miteinander verbunden. Das Verbindungselement 111 verbindet die beiden Endstücke 104, 104' bzw. die beiden Teile des Griffbereichs 112, 112' vorzugsweise dauerhaft miteinander und ist bevorzugt bereits bei Herstellung der Einführhilfe 101 vorhanden, kann jedoch alternativ die zunächst zusammenhangslos hergestellten Teile 104, 104' bzw. 112, 112' oder auch beide Hälften A, B auch erst später miteinander verbinden.

Am Endstück 104, 104' ist zudem ein weiteres Verbindungselement 110A, 110B in Form eines Rastverschlusses vorgesehen, wobei die zueinander passenden Rastelemente 110A, 110B jeweils an sich entsprechenden Stellen am Endstück 104, 104' vorgesehen sind.

Durch die Einführhilfe 101 verläuft ein durchgängiger Kanal, durch den ein Katheter geführt werden kann. Der Kanal erweitert sich am proximalen Ende des Endstück 104, 104' langsam, um das Einführen des Katheters (nicht dargestellt) in die Einführhilfe 101 zu erleichtern.

Figur 15 zeigt eine perspektivische Ansicht einer alternativen Bauform der zweiten Ausführungsform der Einführhilfe 101. Diese alternative Bauform unterscheidet sich allein durch die Anordnung des Kopfstücks 102, welches vorliegend nicht zweigeteilt sondern als ungeteiltes Rohr ausgeführt ist. Das Kopfstück ist über ein Kopplungselement 109 mit dem zweigeteilten Mittelstück 103, 103' verbunden. Das Kopplungsstück 109 ist in Form einer Manschette vorgesehen, welche den proximalen Teil des Kopfstückes 102 und den distalen Teil des Mittelstücks 103, 103' umfasst. Zur Verbindung von Kopfstück 102, Kopplungsstück 109 und Mittelstück 103, 103' können beispielsweise kraftschlüssige Steckverbindungen oder Gewinde vorgesehen sein.

Somit dient das Kopplungselement 109 gleichzeitig auch als weiteres Verbindungselement der Mittelstückhälften 103, 103'.

Die übrigen Elemente der Einführhilfe 101, insbesondere die Ausgestaltung des Endstücks 104, 104' mit dem weiteren Verbindungselement 110 entspricht der zweiten Ausführungsform gemäß Figur 14.

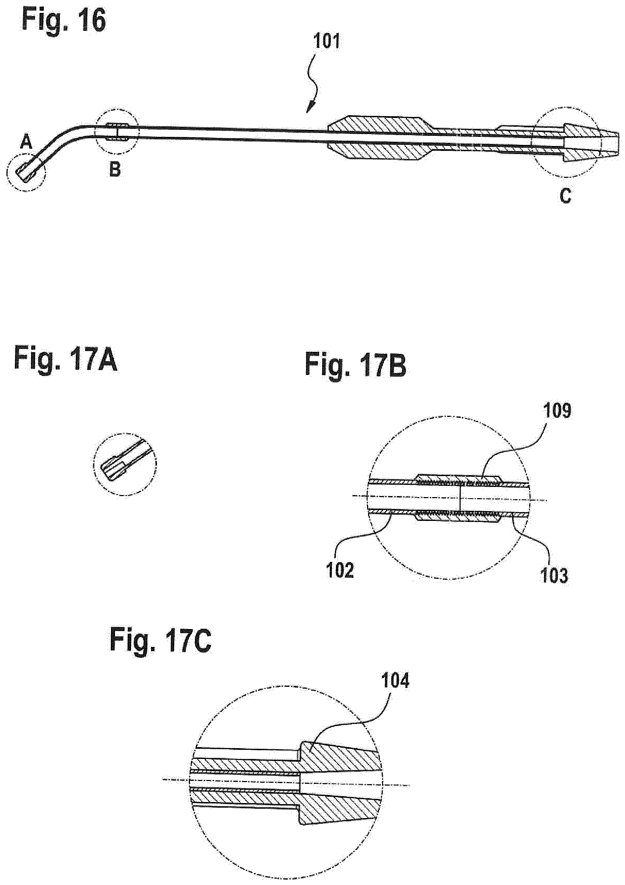

Figur 16 zeigt eine Aufsicht auf die Innenseite einer Hälfte der Einführhilfe 101 gemäß Figur 15 im Längsschnitt. Die einzelnen Elemente entsprechen im Wesentlichen der Figur 14, auf die entsprechend verwiesen wird. Im Unterschied zeigt diese Darstellung der alternativen Bauform insbesondere die Anordnung des Kopplungselements 109, das hier als Manschette um die zueinander angeordneten Enden des Kopf- und Mittelstücks vorgesehen ist.

Figur 17 zeigt in Teilansichten Details bestimmter Abschnitte der alternativen zweiten Ausführungsform gemäß Figuren 15 und 16.

Figur 17A zeigt einen Längsschnitt durch das distale Ende des Kopfstücks.

Figur 17B zeigt das Kopplungselement 109 im Längsschnitt mit den jeweiligen Enden des Kopfstücks 102 und des Mittelstücks 103 Die Verbindung dieser drei Elemente erfolgt durch ein Gewinde, nämlich ein Innengewinde im Kopplungselement 109 und Außengewinde an den entsprechenden Bereichen des Kopfstücks 102 und des Mittelstücks 103.

Figur 17C zeigt schließlich den proximal erweiterten Bereich des Endstücks 104 zur Erleichterung der Einführung eines Katheters (nicht dargestellt) in die Einführhilfe.

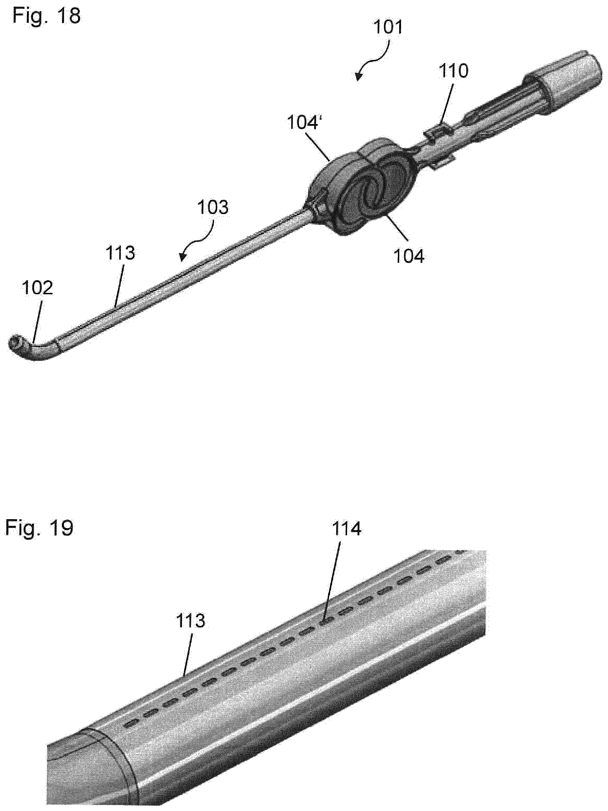

Figur 18 zeigt eine perspektivische Ansicht der alternativen Bauform der zweiten Ausführungsform der Einführhilfe 101 gemäß Figur 15. Zusätzlich umfasst diese Ausführungsform eine Hülle 113, die an dem Kopf- und/oder Mittelstück 102, 103 vorgesehen ist und diesen eng anliegt. Die Hülle 113 gibt den zweiteiligen Kopf- und/oder Mittelstücken 102, 103 zusätzlichen Halt, indem sie die zwei Hälften dieser Bauelemente zusammenhält.

Figur 19 zeigt einen Ausschnitt der Hülle 113 im Detail. Zum Öffnen und Entfernen der Hülle 113 kann eine Perforationslinie 114 vorgesehen sein. Eine solche Perforation dient jedoch vor allem dem Bedienungskomfort und kann problemlos wegfallen. Die Hülle kann vom Benutzer alternative auch durch einen Längsschnitt oder durch Aufreißen entfernt werden.

### Bezugszeichenliste

- 1: Ohrkatheter
- 2: Schirm (A: distaler Abschnitt; B: mittlerer Abschnitt; C: proximaler Abschnitt)
- 3: Katheterschlauch (A: Injektionslumen; B: Druckentlastungslumen; C: distale Öffnung des Injektionslumens; D: distale Öffnung des Druckentlastungslumens; E: Inflationslumen; F: distale Öffnung des Inflationslumens)
- 4: Einwegventil
- 5: Anschlussvorrichtung (Luer-Lock-System)
- 6: Expansionsvorrichtung
- 7: Bedienhilfe
- 8: Einrastvorrichtung
- 9: Verbindung
- 10: proximale Anschlüsse (A: Injektionslumen; B: Druckentlastungslumen; C: Inflationslumen)
- 11: Marker (A: erster Marker; B: zweiter Marker; C: dritter Marker)
- 12: Atraumatische Spitze
- 101: Einführhilfe
- 102: Kopfstück (102 erste Hälfte, 102' zweite Hälfte)
- 103: Mittelstück (103 erste Hälfte, 103' zweite Hälfte)
- 104: Endstück (104 erste Hälfte, 104' zweite Hälfte)
- 105: Trennlinie
- 106: Katheter
- 107: Druckgenerator
- 108: Sicherungselement
- 109: Kopplungselement
- 110: weiteres Verbindungselement (110A, 110B Teile des weiteren Verbindungselements)
- 111: Verbindungselement
- 112: Griffbereich (11 erster Teil, 112' zweiter Teil)
- 113: Hülle
- 114: Perforationslinie
- d: distal
- p: proximal

## Patentansprüche

1. Ohrkatheter zur Einführung in die Ohrtrompete mit einem ersten Schlauch als Katheterschlauch (3) mit mindestens einem ersten Lumen als Injektionslumen (3A) zur Applikation einer Flüssigkeit, mindestens einem zweiten Lumen als Druckentlastungslumen (3B) zur Druckentlastung und einem selbstexpandierbaren Dichtelement (2) zum Verschluss der Ohrtrompete, **dadurch gekennzeichnet, dass** das Dichtelement (2) am distalen Ende des Katheterschlauchs (3) als Schirm vorgesehenen ist und eine Expansionsvorrichtung (6) zur Steuerung der Expansion des Schirms (2) vorgesehen ist und wobei der Schirm (2) selbstexpandierbare Stützelemente und/oder ein selbstexpandierbares Gerüst und eine daran festgelegte Membran umfasst.

2. Ohrkatheter nach Anspruch 1, **dadurch gekennzeichnet, dass** die selbstexpandierbaren Stützelemente und/oder das selbstexpandierbare Gerüst aus einem Formgedächtnismaterial hergestellt sind.

3. Ohrkatheter nach einem der Ansprüche 1 oder 2, **dadurch gekennzeichnet, dass** die Membran aus PTFE oder ePTFE besteht.

4. Ohrkatheter nach einem der vorstehenden Ansprüche, **dadurch gekennzeichnet, dass** die Expansionsvorrichtung (6) als zweiter Schlauch mit einem distalen und einem proximalen Ende vorgesehen ist, der formschlüssig außen um den Katheterschlauch (3) und längsverschiebbar relativ zum Katheterschlauch (2) vorgesehen ist und wobei eine Verschiebung der Expansionsvorrichtung (6) nach proximal den Schirm (3) zur Expansion freigibt und eine Verschiebung nach distal den Schirm (3) komprimiert.

5. Ohrkatheter nach einem der vorstehenden Ansprüche, **dadurch gekennzeichnet, dass** die Expansionsvorrichtung (6) an ihrem proximalen Ende eine Bedienhilfe (7) umfasst.

6. Ohrkatheter nach einem der vorstehenden Ansprüche, **dadurch gekennzeichnet, dass** der Katheterschlauch (3) an seinem proximalen Ende mindestens eine Anschlussvorrichtung (5), insbesondere ein Luer-Lock-System (5) aufweist.

7. Ohrkatheter nach einem der vorstehenden Ansprüche, **dadurch gekennzeichnet, dass** sich das Injektionslumen (3A) und das Druckentlastungslumen (3B) proximal von der Anschlussvorrichtung bis distal des Dichtelements (2) erstrecken.

8. Ohrkatheter nach einem der vorstehenden Ansprüche, **dadurch gekennzeichnet, dass** das distale Ende (12) des Ohrkatheters (1) atraumatisch ausgebildet ist.

9. Ohrkatheter nach einem der vorstehenden Ansprüche, **dadurch gekennzeichnet, dass** zumindest in einem der Lumen des Katheterschlauchs (3), insbesondere dem Injektionslumen (3A) oder dem Druckentlastungslumen (3B), mindestens ein Einwegventil (4) vorgesehen ist.

10. Ohrkatheter nach einem der vorstehenden Ansprüche mit einer Einführhilfe, **dadurch gekennzeichnet, dass** die Einführhilfe (101) ein Kopfstück (102, 102'), ein Mittelstück (103, 103') und ein Endstück (104, 104') umfasst, wobei die Einführhilfe (101) aus einem körperverträglichen Polymer besteht und zumindest durch das Mittelstück (103, 103') und das Endstück (104, 104') jeweils mindestens eine Trennlinie (105) verläuft, entlang derer die Einführhilfe (101) aufgeklappt oder aufgespalten werden kann.

## Claims

1. Ear catheter for insertion into the eustachian tube, including a first tube as catheter tube (3) with at least one first lumen as injection lumen (3A) for application of a liquid, at least one second lumen as pressure relief lumen (3B) for pressure relief, and a self-expandable sealing element (2) for the occlusion of the eustachian tube, **characterized in that** the sealing element (2) is provided in the form of a shield at the distal end of the catheter tube (3) and an expansion device (6) is provided for controlling the expansion of the shield (2), and wherein the shield (2) comprises self-expandable support elements and/or a self-expandable framework and a membrane secured thereto.

2. Ear catheter according to claim 1, **characterized in that** the self-expandable support elements and/or the self-expandable framework are made of a shape memory material.

3. Ear catheter according to any of claims 1 or 2, **characterized in that** the membrane is made of PTFE or ePTFE.

4. Ear catheter according to any one of the preceding claims, **characterized in that** the expansion device (6) is provided as a second tube having a distal and a proximal end, said tube being provided in a form-closed manner externally around the catheter tube (3) and longitudinally displaceable relative to the catheter tube (2), and wherein a displacement of the expansion device (6) in proximal direction causes the shield (3) to be released for expansion and a distal displacement results in a compression of the shield (3).

5. Ear catheter according to any one of the preceding claims, **characterized in that** the expansion device (6) comprises an operating aid (7) arranged at its proximal end.

6. Ear catheter according to any one of the preceding claims, **characterized in that** the catheter tube (3) is provided with at least one connection device (5), in particular a Luer-Lock system (5), which is located at its proximal end.

7. Ear catheter according to any one of the preceding claims, **characterized in that** the injection lumen (3A) and the pressure relief lumen (3B) extend proximally from the connection device to distally of the sealing element (2).

8. Ear catheter according to any one of the preceding claims, **characterized in that** the distal end (12) of the ear catheter (1) is designed so as to be atraumatic.

9. Ear catheter according to any one of the preceding claims, **characterized in that** at least one one-way valve (4) is provided in at least one of the lumens of the catheter tube (3), in particular in the injection lumen (3A) or in the pressure relief lumen (3B).

10. Ear catheter according to any one of claims provided with an insertion aid, **characterized in that** the insertion aid (101) comprises a head piece (102, 102'), a middle piece (103, 103') and an end piece (104, 104'), wherein the insertion aid (101) consists of a biocompatible polymer, and at least one separator line (105) extends at least through the middle piece (103, 103') and the end piece (104, 104') each, which enables the insertion aid (101) to be unfolded or split open along said line.

## Revendications

1. Cathéter auriculaire destiné à être introduit dans la trompe d'Eustache, comprenant un premier tube servant de tube de cathéter (3) avec au moins une première lumière servant de lumière d'injection (3A) pour l'application d'un liquide, au moins une deuxième lumière servant de lumière de décompression (3B) pour la décompression et un élément d'étanchéité auto-expansible (2) pour fermer la trompe d'Eustache, **caractérisé en ce que** l'élément d'étanchéité (2) est prévu comme écran à l'extrémité distale du tube de cathéter (3) et qu'un dispositif d'expansion (6) est prévu pour commander l'expansion du écran (2), l'écran (2) comprenant des éléments de support auto-expansibles et/ou une armature auto-expansible et une membrane fixée à celle-ci.

2. Cathéter auriculaire selon la revendication 1, **caractérisé en ce que** les éléments de support auto-expansibles et/ou l'armature auto-expansible sont fabriqués à partir d'un matériau à mémoire de forme.

3. Cathéter auriculaire selon l'une des revendications 1 ou 2, **caractérisé en ce que** la membrane est en PTFE ou en ePTFE.

4. Cathéter auriculaire selon l'une des revendications précédentes, **caractérisé en ce que** le dispositif d'expansion (6) est prévu sous la forme d'un deuxième tube avec une extrémité distale et une extrémité proximale, qui est prévu pour s'adapter parfaitement à l'extérieur du tube du cathéter (3) et de manière à pouvoir se déplacer longitudinalement par rapport au tube de cathéter (2), un déplacement du dispositif d'expansion (6) vers la partie proximale libérant l'écran (3) pour l'expansion et un déplacement vers la partie distale comprimant l'écran (3).

5. Cathéter auriculaire selon l'une des revendications précédentes, **caractérisé en ce que** le dispositif d'expansion (6) comprend à son extrémité proximale une aide à la manipulation (7).

6. Cathéter auriculaire selon l'une des revendications précédentes, **caractérisé en ce que** le tube du cathéter (3) comporte à son extrémité proximale au moins un dispositif de raccordement (5), en particulier un système Luer-Lock (5).

7. Cathéter auriculaire selon l'une des revendications précédentes, **caractérisé en ce que** la lumière d'injection (3A) et la lumière de décompression (3B) s'étendent de manière proximale depuis le dispositif de raccordement jusqu'à une position distale par rapport à l'élément d'étanchéité (2).

8. Cathéter auriculaire selon l'une des revendications précédentes, **caractérisé en ce que** l'extrémité distale (12) du cathéter auriculaire (1) est conçue de manière atraumatique.

9. Cathéter auriculaire selon l'une des revendications précédentes, **caractérisé en ce qu'**au moins une valve unidirectionnelle (4) est prévue dans au moins l'une des lumières du tube cathéter (3), en particulier la lumière d'injection (3A) ou la lumière de décompression (3B).

10. Cathéter auriculaire selon l'une des revendications précédentes, avec un dispositif d'insertion, **caractérisé en ce que** le dispositif d'insertion (101) comprend une pièce de tête (102, 102'), une pièce centrale (103, 103') et une pièce d'extrémité (104, 104'), le dispositif d'insertion (101) étant constitué d'un polymère biocompatible et au moins une ligne de séparation (105) s'étendant au moins à travers la partie centrale (103, 103') et la partie terminale (104, 104'), le long de laquelle l'aide à l'insertion (101) peut être dépliée ou fendue.
